# EUROPEAN PATENT APPLICATION

(11) **EP 4 098 243 A1**
(43) Date of publication of application: **07.12.2022**
(21) Application number: 21176923.7
(22) Date of filing: 31.05.2021
(51) Int. Cl.: A61K 8/26, A61K 8/31, A61K 8/36, A61K 8/92, A61Q 9/02, B26B 21/44

(54) **SOLID SHAVING AID COMPOSITION**

(71) Applicant: BIC Violex Single Member S.A., 14569 Anoixi (GR)
(72) Inventor: Zanidaki, Maria-Thiresia, 145 69 Anoixi (GR); Vlachos, Georgios, 145 69 Anoixi (GR)
(74) Representative: Peterreins Schley

(57) **Abstract**

The present disclosure to a razor cartridge comprising a housing having a front edge and a rear edge. The housing comprises one or more shaving blades disposed between the front edge and the rear edge. At least one shaving bar is disposed in front of the blade(s) and/or rear of the blade(s). The shaving bar comprises a solid shaving aid composition. The solid shaving aid composition may comprise, relative to the total weight of the solid shaving aid composition: about 10 wt.-% to about 75 wt.-% of a soap base comprising one or more fatty acid salts having from about 8 to about 24 carbon atoms and optionally one or more fatty acids having from about 8 to about 24 carbon atoms, about 0.2 wt.-% to about 5.0 wt.-% of one or more waxes, and about 0.2 wt.-% to about 5.0 wt.-% of one or more phyllosilicates; wherein the combined amount by weight of the one or more waxes and the one or more phyllosilicates is at least about 0.5 wt.-%, in particular about 2.0 to about 7.0 wt.-%.

## Description

### Technical Field

The present invention relates to the field of razor cartridges. More specifically, the present invention relates to razor cartridges comprising solid shaving aid compositions.

### Background

Razors generally include a handle and a razor cartridge fixedly or releasably attached to the handle. The razor cartridge includes at least one blade for shaving hair. The razor cartridge may also include a solid shaving aid composition, often in the shape of a shaving aid bar extending parallel to at least one blade. The user holds the handle and repeatedly moves the razor across an area of the body to be shaved, e.g., the face, until hair is removed from the surface of the body. The shaving aid bar may function to aid in shaving and to decrease or prevent skin irritation due to the repeated exposure of the skin to sharp razor blades.

Razor cartridges comprising such solid shaving aid compositions are known in the art, for instance from WO 2016/162080 A1. However, while successful in aiding with comfortable and simplified shaving, there is still room for improvement. For instance, current products may still have limited durability, i.e. sufficiently long acceptance by the user.

### Summary

Following diligent research, it was found that a new formulation based on fatty acid salt soap base is generally advantageous in providing razor cartridges having improved durability when provided with a combination of wax and phyllosilicates.

According to a first aspect, the present disclosure relates to a razor cartridge comprising a housing having a front edge and a rear edge. The housing comprises one or more shaving blades disposed between the front edge and the rear edge. At least one shaving bar is disposed in front of the blade(s) and/or rear of the blade(s). The shaving bar comprises a solid shaving aid composition. The solid shaving aid composition may comprise, relative to the total weight of the solid shaving aid composition: about 10 wt.-% to about 75 wt.-% of a soap base consisting of one or more fatty acid salts having from about 8 to about 24 carbon atoms and optionally one or more fatty acids having from about 8 to about 24 carbon atoms, about 0.2 wt.-% to about 5.0 wt.-% of one or more waxes, and about 0.2 wt.-% to about 5.0 wt.-% of one or more phyllosilicates; wherein the combined amount by weight of the one or more waxes and the one or more phyllosilicates is at least about 0.5 wt.-%, in particular about 2.0 to about 7.0 wt.-%.

In some embodiments, the one or more phyllosilicates may comprise a negatively charged phyllosilicate, in particular a smectite.

In some embodiments, the one or more phyllosilicates may comprise a montmorillonite, a beidellite, a nontronite, a saponite or a hectorite.

In some embodiments, the one or more phyllosilicates may have an idealized formula of M+x A12Si4-xAlxO10OH2 and optionally crystal water, wherein M+ represents interlayer earth alkali cations, in particular Na+ and/or Li+ and wherein x is between about 0.2 to about 0.6.

In some embodiments, the composition may comprise between about 0.5 to about 2.5 wt.-% of the one or more phyllosilicates, relative to the total weight of the solid shaving aid composition.

In some embodiments, the soap base may comprise one or more monovalent linear or branched saturated or unsaturated carboxylic acid salts having between about 8 and about 24 carbon atoms, in particular between about 10 and about 20 carbon atoms; and optionally one or more monovalent linear or branched saturated or unsaturated carboxylic acids having between about 8 and about 24 carbon atoms, in particular between about 10 and about 20 carbon atoms.

In some embodiments, the soap base may comprise one or more salts of cocoate, stearate, palmitate, laurate, myristate.

In some embodiments, the solid shaving aid composition may comprise the one or more fatty acid salts and, if present, the fatty acids in an amount of between about 10 wt.-% to about 50 wt.-%, more specifically between about 15 to about 45 wt.-%, and in particular between about 17 wt.-% and about 39 wt.-%, relative to the total weight of the solid shaving aid composition.

In some embodiments, the one or more of the waxes may have a solubility in water at about 25°C of less than about 1000 mg/L.

In some embodiments, the one or more of the waxes may have a melting point of at least about 40°C.

In some embodiments, the one or more of the waxes may comprise a hydrocarbon-based wax, in particular a paraffin.

In some embodiments, the one or more of the waxes may comprise an ester wax, in particular a carnauba wax.

In some embodiments, the solid shaving aid composition may comprise between about 1.0 wt.-% and about 5.0 wt.-%, and in particular between about 2.5 and about 4 wt.-%, of the one or more waxes, relative to the total weight of the solid shaving aid composition.

In some embodiments, the solid shaving composition may be shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

In another aspect, the present disclosure relates to a solid shaving aid composition having a composition as defined for the first aspect of the present disclosure.

### Detailed Description

Hereinafter, a detailed description will be given of the present disclosure. The terms or words used in the description and the claims of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

It was found that a new formulation based on fatty acid salts is generally advantageous in providing razor cartridges having good durability when the formulation is provided with a combination of waxes and phyllosilicates..

Without wishing to be bound by theory, it is believed that the combination of waxes and phyllosilicates may contribute to durability of the solid shaving aid. In use, the fatty acid salt soap base may form an emulsion together with water added during the shaving action. This emulsification is desirable since it may provide a glide film for the razor cartridge to move on across the skin and may clean and moisturize the skin. However, excessive emulsification of the solid shaving aid may be detrimental to the durability since the shaving aid is consumed so fast that it cannot provide its full benefits at the end of the lifetime of the razor cartridge. Without wishing to be bound by theory, the phyllosilicates may counter excessive emulsification while not being overly detrimental to the overall performance of the shaving aid by virtue of the layered structure of the phyllosilicates: Phyllosilicates are layered minerals and said layers may absorb water in the interstitial space between these layers. The hydrated phyllosilicates and the water may exhibit a high degree of cohesion due to formation of hydrogen bridge bonds and may contribute to the thickening of the emulsion formed by the solid shaving aid. Again without wishing to be bound by theory, it is believed that a thicker emulsion initially formed on the surface of the solid shaving aid may help in preventing excessive solubilization and premature loss of the solid shaving aid. Finally, again without wishing to be bound by theory, it is believed that the waxes may further contribute to the aforementioned effect: Waxes may act as film formers on the solid shaving aid and, due to their limited solubility, may help in further reducing premature dissolution of the solid shaving aid.

Accordingly, in a first aspect, the present disclosure relates to a razor cartridge comprising a housing having a front edge and a rear edge. The housing comprises one or more shaving blades disposed between the front edge and the rear edge. At least one shaving bar is disposed in front of the blade(s) and/or rear of the blade(s). The shaving bar comprises a solid shaving aid composition. The solid shaving aid composition may comprise, relative to the total weight of the solid shaving aid composition: about 10 wt.-% to about 75 wt.-% of a soap base consisting one or more fatty acid salts having from about 8 to about 24 carbon atoms and optionally one or more fatty acids having from about 8 to about 24 carbon atoms, about 0.2 wt.-% to about 5.0 wt.-% of one or more waxes, and about 0.2 wt.-% to about 5.0 wt.-% of one or more phyllosilicates; wherein the combined amount by weight of the one or more waxes and the one or more phyllosilicates is at least about 0.5 wt.-%, in particular about 2.0 to about 7.0 wt.-%.

When referring to the soap base in the present disclosure, it should be understood that this is referring to the sum of the aforementioned fatty acid salts and fatty acids as a (major) component of the soap solid shaving aid. Additionally or alternatively, the soap base may be defined as the total summed-up amount of the aforementioned fatty acid salts and, if present, fatty acids.

The shaving bar is not particularly limited and can have any suitable shape. Typically, it is in the form of at least one strip or another elongated body which is arranged substantially parallel to the shaving blades. The shaving bar comprises the shaving aid composition. The shaving aid composition is solid for the present purposes, i.e. solid in the sense that the shaving aid composition retains its shape once applied to the cartridge and does not migrate or "run off under normal storage conditions (e.g. temperature of up to about 25°C and a relative humidity of up to about 60%) and/or intended use conditions. However, it should be understood that the solid state of the shaving aid composition is not particularly limited and also includes wax-like characteristics. The shaving aid composition may be solid at room temperature, e.g. at about 25°C.

As mentioned before, the solid shaving aid composition may comprise phyllosilicates in a content of about 0.2 wt.-% to about 5.0 wt.-%, relative to the total weight of the solid shaving aid composition. In some embodiments the one or more phyllosilicates may comprise a negatively charged phyllosilicate, in particular a smectite. The smectite may comprise layers, wherein the layers may carry an overall negative charge. In its entirety, the overall layer structure of the phyllosilicate may be negatively charged. The negative charge may be counterbalanced by the presence of interlayer cations, in particular earth alkali cations.

In some embodiments the one or more phyllosilicates may comprise a montmorillonite, a beidellite, a nontronite, a saponite or a hectorite.

In some embodiments the one or more phyllosilicates may have an idealized formula of M⁺ₓ Al₂Si₄₋ₓAlₓO₁₀OH₂ and optionally crystal water, wherein M⁺ represents interlayer earth alkali cations, in particular Na⁺ and/or Li⁺ and wherein x is between about 0.2 to about 0.6.

In some embodiments the composition may comprise between about 0.5 to about 2.5 wt.-% of the one or more phyllosilicates, relative to the total weight of the solid shaving aid composition.

As mentioned before, the phyllosilicates may comprise layers and absorb water in the interlayer space between the layers. The hydrated phyllosilicates may contribute to the thickening of the emulsion formed by the fatty acid salt soap base.

In some embodiments the composition may comprise as fatty acid salts one or more monovalent linear or branched saturated or unsaturated fatty acid salts having between about 8 and about 24 carbon atoms, in particular between about 10 and about 20 carbon atoms; and optionally as fatty acids one or more monovalent linear or branched saturated or unsaturated fatty acids having between about 8 and about 24 carbon atoms, in particular between about 10 and about 20 carbon atoms. Non-limiting examples of such fatty acid salts include cocoate, stearate, palmitate, laurate, myristate.

In some embodiments the solid shaving aid composition may comprise the one or more fatty acid salts and, if present, the one or more fatty acids in an amount of between about 10 wt.-% to about 50 wt.-%, more specifically between about 15 to about 45 wt.-%, and in particular between about 17 wt.-% and about 39 wt.-%, relative to the total weight of the solid shaving aid composition.

The fatty acid salts may be partly substituted by fatty acids, e.g. from about 0.1 to about 20 wt-%, in particular from about 0.1 to about 10 wt-%, in particular from about 0.1 to about 5 wt.-%, relative to the total weight of the fatty acid salts.

In some embodiments, it may be advantageous that the soap base consists of fatty acid salts, i.e. no fatty acids are present and/or added.

As mentioned before, the shaving aid composition may comprise one or more waxes in a content of about 0.2 wt.-% to about 5.0 wt.-% of one or more waxes The term "wax," in the present disclosure is intended to be used as is well-established in the field of cosmetics and is, in particular, meant to refer to a lipophilic (fatty) compound that is solid at e.g. room temperature (e.g. about 25 °C) with a reversible solid/liquid change of state. The method of measuring the melting point of the wax is not particularly limited and may be measured using a differential scanning calorimeter (DSC), for example the calorimeter sold under the name DSC 3 by the company Mettler Toledo.

In some embodiments, the one or more waxes may have a melting point of at least about 40°C. A higher melting point of the one or waxes may facilitate the aforementioned film-forming capabilities of the composition and may facilitate retaining the effect on the warm skin of the user or when in contact to warm water. In some embodiments, the wax may have a melting point of at least about 40°C, more specifically at least about 42°C and in particular at least about 45°C. In some embodiments, the wax may have a melting point range of between about 40°C and about 120°C, more specifically between about 42°C and about 100°C, and in particular between about 45°C and about 80°C.

In some embodiments, the wax may have a solubility in water at about 25°C of less than about 1000 mg/L, more specifically a solubility of less than 100 mg/L or less than about 10 mg/L, and in particular a solubility of less than about 1 mg/L. In some embodiments, the wax may be substantially insoluble or insoluble in water at about 25 °C. The method of determining the solubility of the wax is not particularly limited and may be performed by any suitable means, for instance using a USP Dissolution Apparatus 2 (paddle type). A lower water solubility may also facilitate the aforementioned film-forming capabilities of the composition.

Examples of waxes include the following:

### a) Hydrocarbon-based wax

In some embodiments, the wax may be a hydrocarbon-based wax. The term "hydrocarbon-based wax" is intended to refer to a wax formed essentially from, or even constituted by, carbon and hydrogen atoms, and possibly oxygen and nitrogen atoms, and not containing any silicon or fluorine atoms. It may contain functional groups such as alcohol, ether, amine and/or amide groups.

In some embodiments one or more of the waxes may comprise a hydrocarbon-based wax, in particular a paraffin.

In some embodiments, it may be advantageous that the hydrocarbon-based wax is a co-surfactant. The term "co-surfactant" in the present disclosure is intended to be used as is well-established in the field of cosmetics and is, in particular, meant to refer to a second (weaker) surfactant that is used in improve or contribute the performance of a primary surfactant (in this case: the anionic surfactants, in particular the fatty acid salts). A weaker co-surfactant has a lower surfactant effectiveness relative to the anionic surfactant when tested under otherwise identical conditions. Suitable testing conditions are well-known in the art. An exemplary and convenient measure of surfactant efficiency includes the amount of surfactant needed to reduce the surface tension by 20 mN/m at a surface age of 0.1 s, as disclosed in Miller, Tenside Surfactants Detergents 42(4):204-209; 2005, the disclosure of which is incorporated herein by reference. However, other tests may be used as well.

One way in which a co-surfactant may improve or contribute the performance of a primary surfactant is that it facilitates the emulsification provided by the anionic surfactant(s), in particular the fatty acids and fatty acid salts, i.e. it acts as an emulsion stabilizer.

In context of the aforementioned it should be understood that the present disclosure does not intend to distinguish between tensides, surfactants, emulsifiers and emollients. All these compounds are endowed with a certain degree of surface-active activity and are used interchangeably unless expressly stated otherwise.

In some embodiments one or more of the waxes may comprise a co-surfactant, in particular a hydroxylated hydrocarbon-based wax. In some embodiments, in particular in cases where the hydrocarbon-based wax is a co-surfactant, it may be particularly advantageous that the hydrocarbon-based wax comprises a linear or branched, saturated or unsaturated, monovalent or multivalent hydrocarbyl alcohol having between about 8 and about 32carbon atoms, specifically between about 10 and about 28 carbon atoms, and in particular between about 12 about 24 carbon atoms. More specifically, the wax may comprise an (in particular a linear) mono- or divalent alkyl alcohol having between about 8 and about 32 carbon atoms, specifically between about 10 and about 28 carbon atoms, and in particular between about 12 about 24 carbon atoms. In some embodiments, the wax may comprise a mono- or divalent fatty alcohol, in particular cetyl alcohol, stearyl alcohol, behenyl alcohol, or mixtures thereof such as cetearyl alcohol. A commercial example includes Lanette O, sold by BASF SE, Germany. A fatty alcohol is the aforementioned sense may in particular be a monovalent linear alkyl alcohol having between about 8 and about 24 carbon atoms.

### b) Ester wax

In some embodiments the wax may comprise an ester wax, in particular a hydrocarbon-based ester wax. The term "ester wax" in the present disclosure is intended to be used as is well-established in the field of cosmetics and is, in particular, meant to refer to a wax comprising at least one ester group.

In some embodiments, the ester wax is an ester of a C₈₋₄₀-hydrocarbyl carboxylic acid, which may optionally be substituted with one or more heteroatoms such as O, N, S or P, with a C₂-C₈ polyol having between 2 and 8 hydroxyl groups. In some embodiments, the C₈-C₄₀-hydrocarbyl carboxylic acid may comprise at least one saturated or unsaturated C₁₀-C₃₂-moiety, more specifically at least one saturated or unsaturated C₁₂-C₂₆-moiety, and in particular at least one saturated or unsaturated C₁₄-C₂₄-moiety. In some embodiments, the C₂-C₈ polyol has between 2 and 6, between 2 and 4, between 3 and 6, or between 3 and 5 hydroxyl groups. In some embodiments, the ester wax is a monoester, a diester, a triester or a tetraester, and in particular a monoester or diester.

In some embodiments, it may be advantageous that the ester wax still comprises hydroxyl groups, i.e. that the ester is (formally) formed by reaction of less C₈-C₄₀-hydrocarbyl carboxylic acids than the number of hydroxyl groups in the C₂-C₈ polyol and/or that the C₈-C₄₀-hydrocarbyl carboxylic acids are substituted with hydroxyl groups. In some embodiments, it may thus be advantageous that the ester wax comprises one, two, three or four (free) hydroxyl groups. The remaining hydroxyl groups may have the advantage of providing co-surfactant performance to the ester wax.

In some embodiments, the ester wax is an ester of a C₈-C₄₀-hydrocarbyl alcohol, which may optionally be substituted with one or more heteroatoms such as O, N, S or P, with a C₂-C₈ carboxylic acid having between 2 and 6 carboxylic acid groups. In some embodiments, the C₈-C₄₀-hydrocarbyl alcohol may comprise at least one saturated or unsaturated C₁₀-C₃₂-moiety, more specifically at least one saturated or unsaturated C₁₂-C₂₆-moiety, and in particular at least one saturated or unsaturated C₁₄-C₂₄-moiety. In some embodiments, the C₂-C₈ carboxylic acid has between 2 and 6, between 2 and 4, between 3 and 6, or between 3 and 5 carboxylic acid groups. In some embodiments, the ester wax is a mono ester, a diester, a triester or a tetraester.

In some embodiments, it may be advantageous that the ester wax still comprises hydroxyl groups, i.e. that the ester is (formally) formed by reaction of more C₈-C₄₀-hydrocarbyl alcohols than the number of carboxylic acid groups in the C₂-C₈ carboxylic acid and/or that the C₈-C₄₀-hydrocarbyl alcohols are substituted with hydroxyl groups. In some embodiments, it may thus be advantageous that the ester wax comprises one, two, three or four (free) hydroxyl groups. The remaining hydroxyl groups may have the advantage of providing co-surfactant performance to the ester wax.

In some embodiments, it may be advantageous that the ester wax comprises an ester of a fatty alcohol and/or an ester of a polyol. In some embodiments, the fatty alcohol is as defined above or, alternatively, a C₈-C₄₀-hydrocarbyl alcohol. In some embodiments, the polyol is a C₂-C₈ polyol, more specifically glycol or glycerol and in particular glycerol. In both cases, the carboxy-group may be formed by a fatty acid. A fatty acid is this sense may in particular be a monovalent linear carboxylic acid having between about 8 and about 24 carbon atoms.

Non-limiting examples of ester waxes include:
i) beeswax which is a mixture of ester waxes comprising as its main constituents palmitate, palmitoleate, and oleate esters of long-chain (30-32 carbons) aliphatic alcohols, with the ratio of triacontanyl palmitate CH₃(CH₂)₂₉O-CO-(CH₂)₁₄CH₃ to cerotic acid CH₃(CH₂)₂₄COOH, the two principal constituents, being about 6:1.
ii) carnauba wax.
iii) mono- and di-C₈-C₄₀-hydrocarbyl esters of glycol or glycerol such as glyceryl stearate, glyceryl distearate, glycol stearate and glycol distearate. Such ester waxes are available from SABO S.p.A, Italy.
iv) C₂₀-C₄₀ alkyl (hydroxystearyloxy)stearates. Such waxes are sold, for example, under the trade names "Kester Wax K 82 P", "Hydroxypolyester K 82 P", "Kester Wax K 80 P", or "Kester Wax K82H" by the company Koster Keunen BV, Netherlands.
v) esters of ethylene glycol and montanic acid (octacosanoic acid). Such waxes are sold, for example, under the trade name "Licowax KPS" by the company Clariant AG, Switzerland.
vi) bis(1,1,1-trimethylolpropane) tetrastearate. Such a wax is sold under the name Hest 2T-4S^{®} by the company Heterene Chemical Co, Inc., USA.
vii) waxes obtained by hydrogenation of castor oil esterified with cetyl alcohol. Such waxes are sold under the trade names Phytowax ricin 16L64^{®} and 22L73^{®} by the company Sophim SA, France.

In some embodiments, it may be particularly advantageous that the ester wax is a carnauba wax.

In some embodiments, the wax may be a mixture of waxes.

In some embodiments, the wax comprises a mixture of at least one hydrocarbon-based wax and at least one ester wax.

In some embodiments, the wax comprises a mixture of at least one hydrocarbon-based wax and at least one ester wax, and the relative amount (by weight) of the hydrocarbon-based wax to the amount (by weight) of the ester wax is between about 10:1 to about 1:10, more specifically between about 5:1 to about 1:5, and in particular between about 3:1 to about 1:3.

In some embodiments, the wax comprises a polar wax. For the purposes of the present disclosure, the term "polar wax" denotes a wax comprising at least one polar group. Polar groups are well known to those skilled in the art: they may *i.a.* be selected from hydroxyl, ester, carboxylic acid or amide groups. Such polar waxes are to be differentiated from apolar waxes which are hydrocarbon-based and are substantially free or free of the aforementioned polar groups.

Examples of polar waxes include the aforementioned linear or branched, saturated or unsaturated, monovalent or multivalent hydrocarbyl alcohol having between about 8 and about 32 carbon atoms and the aforementioned ester waxes.

In some embodiments, the wax comprises an apolar wax. Examples of such apolar waxes include polyethylene waxes, microcrystalline waxes, ozokerine and Fisher-Tropsch waxes.

In some embodiments the solid shaving aid composition may comprise between about 1.0 wt.-% and about 5.0 wt.-%, and in particular between about 2.5 and about 4 wt.-%, of the one or more waxes, relative to the total weight of the solid shaving aid composition.

In some embodiments, the solid shaving aid composition may further comprise one or more monovalent alcohols having a molecular weight of less than about 100 g/mol and/or one or more polyols, in particular polyols having a molecular weight of less than about 250 g/mol. In some instances, such compounds may serve as humectants and/or as processing aids (e.g. as solvent for certain ingredients). Some examples of the aforementioned alcohols include methanol, ethanol and the propanols.

In some embodiments, the one or more polyols comprising from about 2 to about 8 carbon atoms may comprise a saturated linear or branched C₂-C₈ polyol having between 1 and about 7, more specifically between about 2 and about 6, hydroxyl groups; or a mixture of several such compounds and non-exhaustive examples include glycol, glycerol, pentaerythritol, sorbitol, dipropylene glycol, propylene glycol, butylene glycol, propanediol, ethyl hexanediol, 1,2-hexanediol, and pentylene glycol. In some embodiments the one or more polyols comprising from about 2 to about 8 carbon atoms may comprise a mixture of glycerol, sorbitol and at least one of propylene glycol, ethylene glycol, diethylene glycol, dipropylene glycol, and methylpropane diol glycol.

In some embodiments, the solid shaving aid composition may comprise one or more polyols comprising from about 2 to about 8 carbon atoms in an amount of between about 10 wt.-% and about 60 wt.-%, more specifically between about 10 wt.-% and about 55 wt.-% relative to the total weight of the solid shaving aid composition.

In some embodiments, the one or more polyols comprising from about 12 to about 18 carbon atoms may comprise a saturated C₁₂-C₁₈ polyol having between 6 and about 18, more specifically between about 8 and about 14, hydroxyl groups; or a mixture of several such compounds. Non-exhaustive examples of polyols comprising from about 12 to about 18 carbon atoms include disaccharides and trisaccharides such as sucrose, saccharose, lactose and maltose.

In some embodiments, the solid shaving aid composition may comprise one or more polyols comprising from about 12 to about 18 carbon atoms in an amount of between about 1 wt.-% and about 20 wt.-%, more specifically between about 2 wt.-% and about 10 wt.-%, relative to the total weight of the solid shaving aid composition.

The one or more polyols may act as moisturizers. The one or more polyols moisturizing properties may also further contribute to the thickening of the emulsion. Additionally, the one or more polyols may act as an (additional) solvent, which may facilitate production of the solid shaving aid.

In some embodiments, the one or more sulfur-containing anionic surfactant may comprise anionic surfactants selected from alkyl sulfates, sulfosuccinates, alkyl benzene sulfanate, acyl methyl taurates, isethionates, monoglyceride sulfates and ether sulfanates. In particular, the one or more sulfur-containing anionic surfactant may have between about 8 and about 24 carbon atoms, in particular between about 10 and about 20 carbon atoms. The one or more anionic surfactants may provide cleansing power and contribute a (slight) foaming ability, if desirable.

In some embodiments, the solid shaving aid composition may comprise the one or more anionic surfactants in an amount of between about 0.5 wt.-% and about 20 wt.-%, more specifically between about 1.0 wt.-% and about 15 wt.-%, relative to the total weight of the solid shaving aid composition.

The solid shaving aid may comprise one or more butters. For the purpose of the present invention, the term "butter" is intended to mean a lipophilic fatty compound or composition with a reversible solid/liquid change of state, comprising, in its pure form and at a temperature of about 25°C, a liquid and a solid fraction. In other words, the starting melting point of the butter can be less than about 25°C. The liquid fraction of the butter measured at about 25°C can represent between about 9 wt.-% and about 97 wt.-% of the compound, more specifically about 15 wt.-% and about 85 wt.-%, and in particular between about 40 wt.-% and about 85 wt.-%.

In some embodiments, the butter(s) may have an end melting point of less than about 60°C.

For the purposes of this disclosure, the melting point of a butter may correspond to the temperature of the most endothermic peak observed on thermal analysis (DSC) as described in Standard ISO 1 1357-3:1999. Said melting point may e.g. be measured using a differential scanning calorimeter (DSC), for example the calorimeter sold under the name DSC Q2000 by the company TA Instruments, USA. As regards the measurement of the melting point and the determination of the end melting point, an exemplary sample preparation and measurement protocols may be as follows:
A sample of 5 mg of the butter, preheated to 80°C and withdrawn with magnetic stirring using a spatula that is also heated, is placed in a hermetic aluminium capsule, or crucible. Two tests are performed to ensure the reproducibility of the results. The measurements are performed on the abovementioned calorimeter. The oven is flushed with nitrogen. Cooling is provided by an RCS 90 heat exchanger. The sample is then subjected to the following protocol: it is first of all placed at a temperature of 20°C, and then subjected to a first temperature rise passing from 20°C to 80°C, at a heating rate of 5°C/minute, then is cooled from 80°C to -80°C at a cooling rate of 5°C/minute and finally subjected to a second temperature rise passing from -80°C to 80°C at a heating rate of 5°C/minute. During the second temperature rise, the variation in the difference in power absorbed by the empty crucible and by the crucible containing the sample of butter is measured as a function of the temperature. The melting point of the butter is the value of the temperature corresponding to the top of the peak of the curve representing the variation in the difference in power absorbed as a function of the temperature.

The end melting point corresponds to the temperature at which 95% of the sample has melted.

The liquid fraction by weight of the butter at 25°C is equal to the ratio of the heat of fusion consumed at 25 °C to the heat of fusion of the butter.

The heat of fusion of the butter is the heat consumed in order to transition from the solid state to the liquid state. The heat of fusion of the butter is equal to the integral of the entire melting curve obtained using the abovementioned calorimeter, with a temperature rise of 5 or 10°C/minute, according to Standard ISO 1 1357-3:1999. The heat of fusion of the butter is the amount of energy required to make the butter change from the solid state to the liquid state. It is expressed in J/g.

The heat of fusion consumed at 25°C is the amount of energy absorbed by the sample to change from the solid state to the state that it has at 25°C, consisting of a liquid fraction and a solid fraction.

The liquid fraction of the butter measured at 32°C may represent from 30% to 100% by weight of the butter, more specifically from 50% to 100%, and in particular from 60% to 100% by weight of the compound. When the liquid fraction of the butter measured at 32°C is equal to 100%, the temperature of the end of the melting range of the butter is less than or equal to 32°C.

The liquid fraction of the butter measured at 32°C is equal to the ratio of the heat of fusion consumed at 32°C to the heat of fusion of the butter. The heat of fusion consumed at 32°C is calculated in the same way as the heat of fusion consumed at 23°C.

In some embodiments, the butter may have a hardness of less than or equal to about 6 MPa. As regards the measurement of the butter hardness, an exemplary sample preparation and measurement protocols may be as follows:
The butter is placed in a mould 75 mm in diameter, which is filled to about 75% of its height. In order to overcome the thermal history and to control the crystallization, the mould is placed in a Votsch VC0018 programmable oven, where it is first of all placed at a temperature of 80°C for 60 minutes, then cooled from 80°C to 0°C at a cooling rate of 5°C/minute, then left at the stabilized temperature of 0°C for 60 minutes, and then subjected to a temperature rise passing from 0°C to 20°C, at a heating rate of 5°C/minute, and then left at the stabilized temperature of 20°C for 180 minutes.

The compressive force measurement is taken using a TA/TX2i texturometer from Swantech. The spindle used is chosen according to the texture:
- cylindrical steel spindle 2 mm in diameter for very rigid starting materials;
- cylindrical steel spindle 12 mm in diameter for relatively non-rigid starting materials.

The measurement comprises three steps:
- a first step after automatic detection of the surface of the sample, where the spindle moves at a measuring speed of 0.1 mm/second, and penetrates into the composition according to the invention or the butter to a penetration depth of 0.3 mm, and the software notes the maximum force value reached;
- a second step, known as relaxation, where the spindle remains in this position for one second and where the force is noted after 1 second of relaxation; and finally
- a third step, known as withdrawal, where the spindle returns to its original position at a speed of 1 mm/second, and the withdrawal energy of the probe (negative force) is noted.

The hardness value measured during the first step corresponds to the maximum compressive force measured in newtons divided by the area of the texturometer cylinder expressed in mm² in contact with the butter or the composition according to the invention. The hardness value obtained is expressed in megapascals or MPa.

Additionally or alternatively, a butter may be characterized in the solid state (at about 25°C) by an anisotropic crystal organization, which is visible by X-ray observation. Methods for determining the presence of anisotropic crystal organization within butters are not particularly limited and include XRD and in particular X-ray birefringence imaging (XBI).

In some embodiments, the butter(s) is (are) of plant origin, in particular such as those described in Ullmann's Encyclopedia of Industrial Chemistry ("Fats and Fatty Oils", A. Thomas, Published Online: 15 JUN 2000, DOI: 10.1002/14356007.a10_173, point 13.2.2.2. Shea Butter, Borneo Tallow, and Related Fats (Vegetable Butters)) which are incorporated herein by reference.

In some embodiments, the butter(s) comprise butters selected from the following list: Shea butter, Nilotica shea butter (Butyrospermum parkii), galam butter (Butyrospermum parkii), Borneo butter or fat or tengkawang tallow (Shorea stenoptera), shorea butter, illipe butter, madhuca butter or (Bassia) Madhuca longifolia butter, mowrah butter (Madhuca latifolia), katiau butter (Madhuca mottleyana), phulwara butter (M. butyracea), mango butter (Mangifera indica), murumuru butter (Astrocaryum murumuru), kokum butter (Garcinia indica), ucuuba butter (Virola sebifera), tucuma butter, painya (kpangnan) butter (Pentadesma butyracea), coffee butter (Coffea arabica), apricot butter (Prunus armeniaca), macadamia butter (Macadamia ternifolia), grapeseed butter (Vitis vinifera), avocado butter (Persea gratissima), olive butter (Olea europaea), sweet almond butter (Prunus amygdalus dulcis), cocoa butter (Theobroma cacao) and sunflower butter.

In some embodiments at least one butter may be selected from the group consisting of cocoa butter, mango butter and mixtures thereof. It may be particularly advantageous that at least one butter is a cocoa butter.

In some embodiments the solid shaving aid may comprise a total content of butter(s) of between about 0.2 wt.-% to about 5 wt.-%, more specifically between about 0.5 wt.-% to about 3.5 wt.-%, relative to the total weight of the solid shaving aid composition.

Butters may help in moisturizing dry and irritated skin. Further, butters may provide skin-soothing and regenerative properties. Furthermore, when in contact with warm skin, butters may (partially) melt and provide a film on the skin, which may provide improved glide and maneuverability to the razor.

As mentioned before, in some embodiments the solid shaving aid composition may comprise an oil. Within the present disclosure, the term "oil" refers to C₈-C₅₀-substances that are liquid at room temperature (about 25°C). These oils may be hydrocarbon-based oils of animal, plant, mineral or synthetic origin, silicone oils and/or fluoro oils, and mixtures thereof. The term "hydrocarbon-based oil" refers to an oil mainly containing carbon atoms and hydrogen atoms (e.g. at least about 70 or about 80 atomic wt.-%) .

Specific examples of oils that can be used include:
- hydrocarbon-based oils of animal origin, such as perhydrosqualene (squalane);
- hydrocarbon-based plant oils, such as liquid triglycerides of fatty acids of 4 to 10 carbon atoms, such as heptanoic or octanoic acid triglyceride; coconut oil, sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, and jojoba oil;
- linear or branched hydrocarbons of mineral or synthetic origin, such as liquid paraffin and derivatives thereof, petroleum jelly, polydecenes and hydrogenated polyisobutene, such as parleam;
- synthetic esters and ethers, in particular, of fatty acids, such as the oils of formula R₁₅COOR₁₆ in which R₁₅ represents a fatty acid residue containing from about 7 to about 25 carbon atoms and R₁₆ represents a hydrocarbon-based chain containing from about 3 to about 25 carbon atoms, such as, for example, purcellin oil, isononyl isononanoate, isopropyl myristate, 2-ethylhexyl palmitate, 2-octyldodecyl stearate, 2-octyldodecyl erucate, isostearyl isostearate; hydroxylated esters such as isostearyl lactate, octyl hydroxystearate, octyldodecyl hydroxystearate, diisostearyl malate, triisocetyl citrate, fatty alkyl heptanoates, octanoates or decanoates; polyol esters such as propylene glycol dioctanoate, neopentyl glycol diheptanoate, diethylene glycol diisononanoate; and pentaerythritol esters such as pentaerythrityl tetraisostearate;

In some embodiments the one or more oils may comprise an oil selected from triglycerides and/or triglyceride derivatives and in particular a coconut oil.

In some embodiments, it may be advantageous that the one or more oils comprises a glycerol esterified with three fatty acids. The fatty acids may be as defined above.

In some embodiments, the solid shaving aid composition may comprise, relative to the total weight of the solid shaving aid composition, between about 0.2 wt.-% to about 5 wt.-%, more specifically between about 0.4 wt.-% and about 3 wt.-%, and in particular between about 0.6 wt.-% and about 2.5 wt.-%, of the one or more oils.

Oils may be added to provide a film on the skin, which may improve the glide and maneuverability of the razor. It may be particularly advantageous that the oils comprise a squalene and/or a coconut oil.

In some embodiments, the aforementioned one or more oils may comprise one or more humectant oils. The humectant oils may have a molecular weight of greater than about 500 g/mol, more specifically greater than about 700 g/mol, and in particular greater than about 900 g/mol. In some embodiments the humectant oils may comprise a castor oil derivative, more specifically a castor oil esterified with 1, 2 or 3 dicarboxylic acid(s) having between about 3 and about 8 carbon atoms. In particular, the humectant oil may be castor oil derivative, and in particular castoryl maleate.

In some embodiments, the solid shaving aid composition may comprise, relative to the total weight of the solid shaving aid composition, between about 0.1 wt.-% to about 2 wt.-%, more specifically between about 0.2 wt.-% and about 1 wt.-%, and in particular between about 0.2 wt.-% and about 0.8 wt.-%, of the aforementioned one or more humectant oils having a molecular weight of greater than about 500 g/mol.

In some embodiments, the solid shaving aid composition may comprise, relative to the total weight of the solid shaving aid composition, about 0.1 wt.-% to about 3 wt.-%, more specifically between about 0.2 wt.-% and about 1 wt.-%, and in particular between about 0.2 wt.-% and about 0.8 wt.-%, of the one or more humectant oils having a molecular weight of greater than about 500 g/mol; and, in total, between about 0.2 wt.-% to about 5 wt.-%, more specifically between about 0.4 wt.-% and about 3 wt.-%, and in particular between about 0.6 wt.-% and about 2.5 wt.-%, of the one or more oils.

The humectant oil may improve skin hydration. Further, humectants oils such as castoryl maleate and its derivatives may contribute to providing a film on the skin which may improve gliding and maneuverability of a razor cartridge.

In some embodiments the one or more silicone cross-polymers may comprise a first (di)methicone-based polymer chain which is crosslinked to a second (di)methicone-based polymer chain via at least one hydrophilic oligomer/polymer chain. In some embodiments the first and/or second dimethicone-based polymer chain may be selected from dimethicone and optionally substituted linear or branched saturated or unsaturated C₁-C₂₀ alkyl (di)methicone. In some embodiments the hydrophilic oligomer/polymer chain may be a C₁-C₄-alkyl polyether wherein the C₁-C₃-alkyl residues are optionally substituted with hydroxyl, hydroxyl-C₁-C₄-alkyl, C₁-C₄-alkyl ether groups or hydroxyl-C₁-C₄-alkylether groups, in particular polyethylene glycol, polypropylene glycol, polyglycerol, and polysorbate. In some embodiments the one or more silicone cross-polymer may comprise polyglycerine-modified cross-linked polymers, polyether-modified cross-linked polymers, polyether/alkyl co-modified cross-linked polymers, polyglycerine/alkyl co-modified cross-linked polymers, dimethicone/vinyl dimethicone cross-polymers, dimethicone/phenyl vinyl dimethicone cross-polymers, vinyl dimethicone/lauryl dimethicone cross-polymers, dimethicone crosspolymers, C₃₀₋₄₅- alkyl cetearyl dimethicone cross-polymers, cetearyl dimethicone cross-polymers, in particular dimethicone/vinyl dimethicone cross-polymers, vinyl dimethicone/lauryl dimethicone cross-polymers, vinyl dimethicone/lauryl dimethicone cross-polymers, lauryl dimethicone/polyglycerin cross-polymers, dimethicone/polyglycerin cross-polymers, polyethylene glycol/polypropoylene glycol-dimethicone cross-polymer; and mixtures thereof.

In some embodiments, the solid shaving aid composition may comprise, relative to the total weight of the solid shaving aid composition, between about 0.1 wt.-% and about 5 wt.-% of one or more of the silicone cross-polymers.

The nonionic surfactants may, for example, be chosen from those compounds that are well known per se (see, for example, "Handbook of Surfactants" by M. R. Porter, published by Blackie & Son (Glasgow and London), 1991, pp. 116-178) which are incorporated herein by reference. The nonionic surfactant may in particular be selected from polyethoxylated, polypropoxylated and polyglycerolated fatty acids, alkylphenols, α-diols and alcohols comprising at least one fatty chain comprising, for example, from about 8 to about 18 carbon atoms, it being possible for the number of ethylene oxide or propylene oxide groups to range, for example, from about 2 to about 200 and for the number of glycerol groups to range, for example, from about 2 to about 100.

In some embodiments, the solid shaving aid comprises polyethoxylated, polypropoxylated and polyglycerolated C₈-C₃₀ fatty acid with a polymerization degree of 2 to about 200, more specifically a polyethylene glycolester of C₈-C₃₀ fatty acid, and in particular a polyethylene glycolester of stearic acid with a polymerization degree of 2 to about 200, such as PEG-100 stearate.

The non-ionic surfactants may also be selected from copolymers of ethylene oxide and of propylene oxide, condensates of ethylene oxide and of propylene oxide with C₈-C₃₀-fatty alcohols; polyethoxylated C₈-C₃₀-fatty amines, for example, those comprising from about 2 to about 30 mol of ethylene oxide, polyglycerolated C₈-C₃₀-fatty amides comprising on average from about 1 to about 5 glycerol groups, for example, from about 1.5 to about 4, glycerol groups; polyethoxylated C₈-C₃₀-fatty amines, for example, comprising from 2 to 30 mol of ethylene oxide; oxyethylenated C₈-C₃₀-fatty acid esters of sorbitan comprising from 2 to 30 mol of ethylene oxide; C₈-C₃₀-fatty acid esters of sucrose, C₈-C₃₀-fatty acid esters of polyethylene glycol, C₈-C₃₀-alkylpolyglycosides, and N-C₈-C₃₀-alkylglucamine derivatives.

The non-ionic surfactants may also be selected from alkylpolyglycosides, in particular C₈-C₃₀-alkylpolyglycosides.

In some embodiments. it may be particularly advantageous that the non-ionic surfactant comprises polyglyceryl-4 oleate.

In some embodiments the solid shaving aid composition may further comprise a non-ionic surfactant wherein the non-ionic surfactant is not a wax as defined above, in particular not a co-tenside wax.

In some embodiments the solid shaving aid composition may comprise, relative to the total weight of the solid shaving aid composition, less than about 8 wt.-%, more specifically less than about 4 wt.-%, and in particular less than about 2 wt.-%, of the non-ionic surfactant. In some embodiments the solid shaving aid composition may comprise between about 0.05 wt.-% and about 4.0 wt.-%, of one or more non-ionic surfactants or non-ionic emulsifiers, relative to the total weight of the solid shaving aid composition.

Non-ionic surfactants may act as cleansing agents and may reduce skin irritation.

In some embodiments the solid shaving aid composition may further comprise a number of generally minor optional ingredients which will be discussed below. It should be understood that the below list is not exhaustive and does not exclude the presence of other ingredients not listed below.

In some embodiments the solid shaving aid composition may comprise between about 0.5 wt.-% and about 6 wt.-%, and in particular between about 1.5 wt.-% and about 4 wt.-% of one or more opacifying agents, relative to the total weight of the solid shaving aid composition. In some embodiments the one or more opacifiying agents may comprise a polymeric opacifiying agent, in particular a styrene/acrylate copolymer, and/or an inorganic pigment, in particular titanium dioxide.

In some embodiments the solid shaving aid composition may comprise between about 0.1 wt.-% and about 2.5 wt.-%, and in particular between 0.3 wt.-% and about 1.5 wt.-% of one or more fragrances, relative to the total weight of the solid shaving aid composition.

In some embodiments the solid shaving aid composition may comprise between 0.01 wt.-% and about 4.5 wt.-% of one or more bulking agent, relative to the total weight of the solid shaving aid composition. In some embodiments the bulking agent may comprise sodium chloride.

In some embodiments the solid shaving aid composition may comprise between about 0.3 wt.-% and about 2 wt.-%, and in particular between 0.4 wt.-% and about 1.8 wt.-% of one or more chelating agents, relative to the total weight of the solid shaving aid composition.
In some embodiments the chelating agent comprises a multidentate in particular pentasodium pentetate, tetrasodium etidronate, tetrasodium iminodissucinate, sodium citrate, citric acid and/or mixtures thereof.

In some embodiments the solid shaving aid composition may comprise, relative to the total weight of the solid shaving aid composition, between about 0.01 wt.-% and about 2.0 wt.-%, in particular between about 0.05 wt.-% and about 1.0 wt.-%, of a preservative or mixture of preservatives, in particular an antibacterial agent. Examples include benzyl alcohol, phenoxyethanol and dehydroacetic acid.

In some embodiments the solid shaving aid composition may comprise a cationic surfactant, in particular a cationic surfactant having a positively charged nitrogen-based group, in particular a quaternary ammonium group. In some embodiments the cationic surfactant may be a saturated or unsaturated hydrocarbon-based compound comprising a quaternary ammonium group and having between about 10 and about 40 carbon atoms, specifically between about 12 and about 36 carbon atoms, and in particular between about 14 about 32 carbon atoms.

In some embodiments, the solid shaving aid composition may comprise or consist of the following components wherein the components refer to the components as defined above, relative to the total weight of the solid shaving aid composition:
a) about 10 wt.-% to about 75 wt.-% of a soap base comprising one or more fatty acid salts having from about 8 to about 24 carbon atoms and optionally one or more fatty acids having from about 8 to about 24 carbon atoms;
b) about 0.2 wt.-% to about 5.0 wt.-% of one or more waxes;
c) about 0.2 wt.-% to about 5.0 wt.-% of one or more phyllosilicates; wherein the combined amount by weight of the one or more waxes and the one or more phyllosilicates is at least about 0.5 wt.-%, in particular about 3.0 to about 7.0 wt.-%;
d) optionally about 10 wt.-% to about 60 wt.-%, more specifically about 10 wt.-% and about 55 wt.-%, of one or more polyols comprising from about 2 to about 8 carbon atoms;
e) optionally about 1 wt.-% to about 20 wt.-% of one or more polyols comprising from about 12 to about 18 carbon atoms;
f) optionally about 0.5 wt.-% to about 20 wt.-%, more specifically between 1.0 wt.-% and about 15 wt.-%, of one or more sulfur-containing anionic surfactants;
g) optionally about 15 wt.-% to about 50 wt.-% of water;
h) optionally about 0.2 wt.-% to about 5 wt.-% of one or more butters;
i) optionally about 0.2 wt.-% to about 5 wt.-% of one or more oils, further optionally including about 0.1 wt.-% to about 3 wt.-% of a humectant oil having a molecular weight of greater than about 500 g/mol;
j) optionally about 0.1 wt.-% to about 5 wt.-% of one or more silicone cross-polymers;
k) optionally about 0.5 wt.-% to about 6 wt.-% of one or more opacifying agents;
l) optionally about 0.05 wt.-% to about 4.0 wt.-% of one or more non-ionic surfactants or non-ionic emulsifiers; and
m) optionally about 0.01 wt.-% to about 4.5 wt.-% of one or more bulking agents; and
n) optionally up to about 15 wt.-%, in particular about 0.5 to about 10 wt.-%, of further ingredients, in particular the further ingredients defined above (fragrances, chelating agents, anti-oxidants, preservatives, etc.) but also other non-mentioned ingredients (e.g. colorants, etc.).

In some embodiments, the solid shaving aid composition may comprise 2, 3, 4, 5, 6, 7, 8, 9 or 10 of components d) to n). In some embodiments, the solid shaving aid composition may comprise all components d) to n). In some embodiments, the solid shaving aid composition may consist of components a) to n).

In some embodiments, for the purposes of calculating compositional amounts, and in case of an overlap, the compound in question will be allotted according to the category of compound first mentioned in the detailed description. For instance, glycol distearate may be classified as both wax and opacifying agent, but for the purposes of calculating compositional amounts, it will be considered as a wax since the compound category of waxes is mentioned before the opacifying agents in the detailed description.

In some embodiments the solid shaving aid composition may be configured to last for at least about 8 leg shaves or facial shaves, more specifically at least about 9 leg shaves or facial shaves, and in particular at least about 10 leg shaves or facial shaves. The shaves may also be performed on other body areas, such as underarms, bikini etc.

In some embodiments the solid shaving composition may be shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

The solid shaving aid composition having a composition defined as any combination of the above embodiments, may be used in a multiplicity of facial or leg shaving operations, wherein the facial or leg shaving operations is repeated for at least about 8 times, specifically about 9 times, and in particular at least about 10 times, without replacing the solid shaving aid composition.

In a second aspect, the present disclosure relates to a solid shaving aid composition. The solid shaving aid composition may have a composition as defined for the first aspect of the present disclosure.

In a third aspect, the present disclosure relates to the use of a solid shaving aid composition having a composition defined above in a multiplicity of facial or leg shaving operations, wherein the facial or leg shaving operations is repeated for at least about 8 times, specifically about 9 times, and in particular at least about 10 times, without replacing the solid shaving aid composition.

Specific embodiments according to the third aspect may use any of the solid shaving aid compositions defined for the first aspect of the present disclosure.

### Examples

A first representative composition of the below table 1 was prepared by hot mixing its components.

**Table 1: shaving aid composition 1**

| **Components** | **Amount (wt.-%)** |
|---|---|
| Soap Base (Sodium Stearate, Sodium Laurate, Sodium Myristate, Stearic Acid, Lauric Acid) | 18.9-36.7 |
| Magnesium Aluminium Silicate | 0.5 - 2 |
| Waxes (hydrocarbon waxes, carnauba wax) | 2.5 - 4 |
| Water | 23.2 - 41.9 |
| C2-C8 Polyols (Propylene Glycol, Sorbitol, Glycerin, Dipropylene Glycol) | 14 - 44.7 |
| Sucroce | 2.5 - 8 |
| Sulfur-containing anionic tensides (Sodium Laureth Sulfate, Sodium Lauryl Sulfate, Sodium Cocoyl Isethionate) | 5.4 - 12.1 |
| Butters (mangifera indica (mango) seed butter, theobroma cacao (cocoa) seed butter) | 0.5 - 3 |
| Oils (squalane, castoryl maleate) | 0.5 - 3 |
| Silicone cross-polymer (lauryl dimethicone polyglycerin-3 crosspolymer) | 0.1 - 2 |
| Other Components | 2 - 10 |
| Total | 100 |

A second representative composition of the below table 2 was prepared by hot mixing its components.

**Table 2: shaving aid composition 2**

| **Components** | **Amount (wt.-%)** |
|---|---|
| Soap Base (Sodium Stearate, Sodium Cocoate) | 21.1 -35.9 |
| C2-C8 Polyols (Glycerin, Sorbitol, Propylene Glycol, Dipropylene Glycol) | 18.5 - 44.4 |
| Magnesium Aluminium Silicate | 0.5 - 2 |
| Waxes (hydrocarbon waxes, carnauba wax) | 2.5 - 4 |
| Water | 23.2 - 41.9 |
| Sulfur-containing anionic tensides (Sodium Coco Sulfate, Sodium Cocoyl Isethionate, Sodium Lauryl Sulfate) | 1.7 - 4.7 |
| Oils (squalane, castoryl maleate, coconut oil) | 1 - 2.5 |
| Butters (mangifera indica (mango) seed butter, theobroma cacao (cocoa) seed butter) | 0.5 - 3 |
| Silicone cross-polymer (lauryl dimethicone polyglycerin-3 crosspolymer) | 0.1 - 2 |
| Other components | 2-8 |
| Total | 100 |

Both composition provided good durability and a well-balanced overall shaving performance.

The present disclosure also relates to the following list of embodiments:
1. A razor cartridge comprising:
   a housing having a front edge and a rear edge;
   one or more shaving blades disposed between the front edge and the rear edge; and
   at least one shaving bar disposed in front of the blade(s) and/or rear of the blade(s) which comprises a solid shaving aid composition;
   wherein the solid shaving aid composition comprises, relative to the total weight of the solid shaving aid composition:
      about 10 wt.-% to about 75 wt.-% of a soap base comprising one or more fatty acid salts having from about 8 to about 24 carbon atoms and optionally one or more fatty acids having from about 8 to about 24 carbon atoms,
      about 0.2 wt.-% to about 5.0 wt.-% of one or more waxes, and
      about 0.2 wt.-% to about 5.0 wt.-% of one or more phyllosilicates;
      wherein the combined amount by weight of the one or more waxes and the one or more phyllosilicates is at least about 0.5 wt.-%, in particular about 2.0 to about 7.0 wt.-%.
2. The razor cartridge of clause 1, wherein the one or more phyllosilicates comprises a negatively charged phyllosilicate, in particular a smectite.
3. The razor cartridge of clause 1 or clause 2, wherein the one or more phyllosilicates comprises a montmorillonite, a beidellite, a nontronite, a saponite or a hectorite.
4. The razor cartridge of any one of clauses 1 to 3, wherein the one or more phyllosilicates has an idealized formula of M⁺ₓ Al₂Si₄₋ₓAlₓO₁₀OH₂ and optionally crystal water, wherein M⁺ represents interlayer earth alkali cations, in particular Na⁺ and Li⁺ and wherein x is between about 0.2 to about 0.6.
5. The razor cartridge of any preceding clause, wherein the composition comprises between about 0.5 to about 2.5 wt.-% of the one or more phyllosilicates, relative to the total weight of the solid shaving aid composition.
6. The razor cartridge of any preceding clause, wherein the soap base comprises one or more monovalent linear or branched saturated or unsaturated carboxylic acid salts having between about 8 and about 24 carbon atoms, in particular between about 10 and about 20 carbon atoms; and optionally one or more monovalent linear or branched saturated or unsaturated carboxylic acids having between about 8 and about 24 carbon atoms, in particular between about 10 and about 20 carbon atoms.
7. The razor cartridge of any preceding clause, wherein the soap base comprises one or more salts of cocoate, stearate, palmitate, laurate, myristate.
8. The razor cartridge of any preceding clause, wherein the solid shaving aid composition comprises the one or more fatty acid salts and, if present, fatty acids in an amount of between about 10 wt.-% to about 50 wt.-%, more specifically between about 15 to about 45 wt.-%, and in particular between about 17 wt.-% and about 39 wt.-%, relative to the total weight of the solid shaving aid composition.
9. The razor cartridge of any preceding clause, wherein one or more of the waxes have a solubility in water at about 25°C of less than about 1000 mg/L.
10. The razor cartridge of any preceding clause, wherein one or more of the waxes have a melting point of at least about 40°C.
11. The razor cartridge of any preceding clause, wherein one or more of the waxes comprise a hydrocarbon-based wax, in particular a paraffinic wax.
12. The razor cartridge of any preceding clause, wherein one or more of the waxes comprise an ester wax, in particular a carnauba wax.
13. The razor cartridge of any preceding clause, wherein one or more of the waxes comprise a co-surfactant, in particular a hydroxylated hydrocarbon-based wax.
14. The razor cartridge of any preceding clause, wherein one or more of the waxes comprise a linear or branched, saturated or unsaturated, monovalent or multivalent hydrocarbyl alcohol having between about 8 and about 32 carbon atoms, specifically between about 10 and about 28 carbon atoms, and in particular between about 12 about 24 carbon atoms.
15. The razor cartridge of any preceding clause, wherein one or more of the waxes comprise an alkyl alcohol having between about 8 and about 32 carbon atoms, specifically between about 10 and about 28 carbon atoms, and in particular between about 12 about 24 carbon atoms.
16. The razor cartridge of any preceding clause, wherein one or more of the waxes comprise a mono- or divalent fatty alcohol, in particular cetyl alcohol, stearyl alcohol, behenyl alcohol or mixtures thereof.
17. The razor cartridge of clause 12, wherein the ester wax comprises a hydrocarbon-based ester wax.
18. The razor cartridge of clause 17, wherein the ester wax comprises an ester of a fatty alcohol and/or an ester of a polyol, in particular glycerol.
19. The razor cartridge of any preceding clause, wherein the solid shaving aid composition comprises between about 0.2 wt.-% and about 5.0 wt.-%, and in particular between about 2.5 and about 4 wt.-%, of the one or more waxes, relative to the total weight of the solid shaving aid composition.
20. The razor cartridge of any preceding clause, wherein the solid shaving aid composition comprises one or more following components in the following amounts, relative to the total weight of the solid shaving aid composition:
   about 10 wt.-% to about 60 wt.-% , more specifically about 10 wt.-% and about 55 wt.-% , of one or more polyols comprising from about 2 to about 8 carbon atoms,
   about 1 wt.-% to about 20 wt.-%, more specifically between about 2 wt.-% and about 10 wt.-%, of one or more polyols comprising from about 12 to about 18 carbon atoms,
   about 0.5 wt.-% to about 20 wt.-% of one or more sulfur-containing anionic surfactants, about 15 wt.-% to about 50 wt.-% of water,
   about 0.2 wt.-% to about 5 wt.-% of one or more butters,
   about 0.2 wt.-% to about 5 wt.-% of one or more oils, in particular including about 0.1 wt.-% to about 3 wt.-% of a humectant oil having a molecular weight of greater than about 500 g/mol,
   about 0.1 wt.-% to about 5 wt.-% of one or more silicone cross-polymers,
   about 0.5 wt.-% to about 6 wt.-% of one or more opacifying agents,
   about 0.05 wt.-% to about 4.0 wt.-% of one or more non-ionic surfactants or non-ionic emulsifiers, and
   about 0.01 wt.-% to about 4.5 wt.-% of one or more bulking agents.
21. The razor cartridge of clause 20, wherein the solid shaving aid composition comprises more than 4, more than 5, more than 6, more than 7 and in particular all components listed in clause 20.
22. The razor cartridge of any one of clauses 20 to 21, wherein the one or more polyols comprising from about 2 to about 8 carbon atoms comprises a saturated linear or branched C₂-C₈ polyol having between 1 and about 7, more specifically between about 2 and about 6, hydroxyl groups; or a mixture of several such compounds.
23. The razor cartridge of any one of clauses 20 to 22, wherein the one or more polyols comprising from about 2 to about 8 carbon atoms comprises a mixture of glycerol, sorbitol and at least one of propylene glycol, ethylene glycol, diethylene glycol, dipropylene glycol, and methylpropane diol glycol.
24. The razor cartridge of any one of clauses 20 to 23, wherein the solid shaving aid composition comprises one or more polyols comprising from about 2 to about 8 carbon atoms in an amount of between about 10 wt.-% and about 60 wt.-%, relative to the total weight of the solid shaving aid composition.
25. The razor cartridge of any one of clauses 20 to 24, wherein the one or more polyols comprising from about 12 to about 18 carbon atoms comprises a saturated C₁₂-C₁₈ polyol having between 6 and about 18, more specifically between about 8 and about 14, hydroxyl groups; or a mixture of several such compounds.
26. The razor cartridge of any one of clauses 20 to 25, wherein the one or more polyols comprising from about 12 to about 18 carbon atoms comprises a disaccharide and/or a trisaccharide, in particular any of or any combination of sucrose, saccharose, lactose and maltose.
27. The razor cartridge of any one of clauses 20 to 26, wherein the solid shaving aid composition comprises one or more polyols comprising from about 12 to about 18 carbon atoms in an amount of between about 1 wt.-% and about 20 wt.-%, more specifically of between about 2 wt.-% and about 10 wt.-%, relative to the total weight of the solid shaving aid composition.
28. The razor cartridge of any one of clauses 20 to 27, wherein the one or more sulfur-containing anionic surfactant comprises anionic surfactants selected from alkyl sulfates, sulfosuccinates, alkyl benzene sulfanate, acyl methyl taurates, isethionates, monoglyceride sulfates and ether sulfanates.
29. The razor cartridge of any one of clauses 20 to 28, wherein the solid shaving aid composition comprises the one or more anionic surfactants in an amount of between about 1.0 wt.-% and about 15 wt.-%, relative to the total weight of the solid shaving aid composition.
30. The razor cartridge of any one of clauses 20 to 29, wherein at least one butter is selected from the group consisting of cocoa butter, mango butter and mixtures thereof.
31. The razor cartridge of any one of clauses 20 to 30, wherein the solid shaving aid comprises a total content of butter(s) of between about 0.2 wt.-% to about 5 wt.-%, more specifically between about 0.5 wt.-% to about 3.5 wt.-% relative to the total weight of the solid shaving aid composition.
32. The razor cartridge of any one of clauses 20 to 31, wherein the one or more oils comprises an oil selected chosen from triglycerides and/or triglyceride derivatives and in particular a coconut oil.
33. The razor cartridge of any one of clauses 20 to 32, wherein the humectant oil having a molecular weight of greater than about 500 g/mol comprises a castor oil derivative, more specifically a castor oil esterified with 1, 2 or 3 dicarboxylic acid(s) having between about 3 and about 8 carbon atoms, and in particular comprises castoryl maleate.
34. The razor cartridge of any one of clauses 20 to 33, wherein the one or more silicone cross-polymers comprises a first (di)methicone-based polymer chain which is crosslinked to a second (di)methicone-based polymer chain via at least one hydrophilic oligomer/polymer chain.
35. The razor cartridge of clause 34, wherein the first and/or second dimethicone-based polymer chain is selected from dimethicone and optionally substituted linear or branched saturated or unsaturated C₁-C₂₀ alkyl (di)methicone.
36. The razor cartridge of clause 34 or clause 35, wherein the hydrophilic oligomer/polymer chain is a C₁-C₄-alkyl polyether wherein the C₁-C₃-alkyl residues are optionally substituted with hydroxyl, hydroxyl-C₁-C₄-alkyl, C₁-C₄-alkyl ether groups or hydroxyl-C₁-C₄-alkylether groups, in particular polyethylene glycol, polypropylene glycol, polyglycerol, and polysorbate.
37. The razor cartridge of any one of clauses 20 to 36, wherein the one or more silicone cross-polymer comprises polyglycerine-modified cross-linked polymers, polyether-modified cross-linked polymers, polyether/alkyl co-modified cross-linked polymers, polyglycerine/alkyl co-modified cross-linked polymers, dimethicone/vinyl dimethicone cross-polymers, dimethicone/phenyl vinyl dimethicone cross-polymers, vinyl dimethicone/lauryl dimethicone cross-polymers, dimethicone crosspolymers, C₃₀₋₄₅-alkyl cetearyl dimethicone cross-polymers, cetearyl dimethicone cross-polymers, in particular dimethicone/vinyl dimethicone cross-polymers, vinyl dimethicone/lauryl dimethicone cross-polymers, vinyl dimethicone/lauryl dimethicone cross-polymers, lauryl dimethicone/polyglycerin cross-polymers, dimethicone/polyglycerin cross-polymers, polyethylene glycol/polypropoylene glycol-dimethicone cross-polymer; and mixtures thereof.
38. The razor cartridge of any one of clauses 20 to 37, wherein the solid shaving aid composition comprises between about 0.05 wt.-% and about 4.0 wt.-%, of one or more non-ionic surfactants or non-ionic emulsifiers, relative to the total weight of the solid shaving aid composition.
39. The razor cartridge of clause 38, wherein the non-ionic surfactant comprises polyglyceryl-4 oleate.
40. The razor cartridge of any one of clauses 20 to 37, wherein the solid shaving aid composition comprises between 0.01 wt.-% and about 4.5 wt.-% of one or more bulking agent, relative to the total weight of the solid shaving aid composition.
41. The razor cartridge of clause 40, wherein the bulking agent comprises sodium chloride.
42. The razor cartridge of any one of clauses 1 to 32, wherein the solid shaving aid composition comprises between about 1 wt.-% and about 5 wt.-%, and in particular between about 0.5 wt.-% and about 6 wt.-%, in particular 1.5 wt.-% and about 4 wt.-%, of one or more opacifying agents, relative to the total weight of the solid shaving aid composition.
43. The razor cartridge of clause 33, wherein the one or more opacifiying agents comprises a polymeric opacifiying agent, in particular a styrene/acrylate copolymer, and/or an inorganic pigment, in particular titanium dioxide.
44. The razor cartridge of any preceding clause, wherein the solid shaving aid composition comprises between about 0.3 wt.-% and about 2 wt.-%, and in particular between 0.4 wt.-% and about 1.8 wt.-% of one or more chelating agents, relative to the total weight of the solid shaving aid composition.
45. The razor cartridge of clause 44, wherein the chelating agent comprises a multidentate in particular pentasodium pentetate, tetrasodium etidronate, tetrasodium iminodissucinate, citric acid and/or sodium citrate.
46. The razor cartridge of any preceding clause, wherein the solid shaving aid composition comprises between about 0.1 wt.-% and about 2.5 wt.-%, and in particular between 0.3 wt.-% and about 1.5 wt.-% of one or more fragrances, relative to the total weight of the solid shaving aid composition.
47. The razor cartridge of any one of clauses 20 to 46, wherein the one or more oils further comprises a hydrocarbon oil, in particular squalene and/or squalane.
48. The razor cartridge of any one of clauses 1 to 46, wherein the solid shaving composition is shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.
49. A solid shaving aid composition having a composition as defined in any one of claims 1 to 48.
50. The solid shaving composition of cany one of clauses 1 to 48, wherein the solid shaving composition is shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

Although the embodiments of the present disclosure have been disclosed for illustrative purposes, those skilled in the art will appreciate that various modifications and alterations are possible, without departing from the spirit of the present disclosure. It is also to be understood that such modifications and alterations are incorporated in the scope of the present disclosure and the accompanying claims.

## Claims

1. A razor cartridge comprising:
a housing having a front edge and a rear edge;
one or more shaving blades disposed between the front edge and the rear edge; and
at least one shaving bar disposed in front of the blade(s) and/or rear of the blade(s) which comprises a solid shaving aid composition;
wherein the solid shaving aid composition comprises, relative to the total weight of the solid shaving aid composition:
about 10 wt.-% to about 75 wt.-% of a soap base consisting one or more fatty acid salts having from about 8 to about 24 carbon atoms and optionally one or more fatty acids having from about 8 to about 24 carbon atoms,
about 0.2 wt.-% to about 5.0 wt.-% of one or more waxes, and
about 0.2 wt.-% to about 5.0 wt.-% of one or more phyllosilicates;
wherein the combined amount by weight of the one or more waxes and the one or more phyllosilicates is at least about 0.5 wt.-%, in particular about 2.0 to about 7.0 wt.-%.

2. The razor cartridge of claim 1, wherein the one or more phyllosilicates comprises a negatively charged phyllosilicate, in particular a smectite.

3. The razor cartridge of claim 1 or claim 2, wherein the one or more phyllosilicates comprises a montmorillonite, a beidellite, a nontronite, a saponite or a hectorite.

4. The razor cartridge of any one of claims 1 to 3, wherein the one or more phyllosilicates has an idealized formula of M⁺ₓ Al₂Si₄₋ₓAlₓO₁₀OH₂ and optionally crystal water, wherein M⁺ represents interlayer earth alkali cations, in particular Na⁺ and/or Li⁺ and wherein x is between about 0.2 to about 0.6.

5. The razor cartridge of any preceding claim, wherein the composition comprises between about 0.5 to about 2.5 wt.-% of the one or more phyllosilicates, relative to the total weight of the solid shaving aid composition.

6. The razor cartridge of any preceding claim, wherein the soap base comprises one or more monovalent linear or branched saturated or unsaturated carboxylic acid salts having between about 8 and about 24 carbon atoms, in particular between about 10 and about 20 carbon atoms; and optionally one or more monovalent linear or branched saturated or unsaturated carboxylic acids having between about 8 and about 24 carbon atoms, in particular between about 10 and about 20 carbon atoms.

7. The razor cartridge of any preceding claim, wherein the soap base comprises one or more salts of cocoate, stearate, palmitate, laurate, myristate.

8. The razor cartridge of any preceding claim, wherein the solid shaving aid composition comprises the one or more fatty acid salts and, if present, the fatty acids in an amount of between about 10 wt.-% to about 50 wt.-%, more specifically between about 15 to about 45 wt.-%, and in particular between about 17 wt.-% and about 39 wt.-%, relative to the total weight of the solid shaving aid composition.

9. The razor cartridge of any preceding claim, wherein one or more of the waxes have a solubility in water at about 25°C of less than about 1000 mg/L.

10. The razor cartridge of any preceding claim, wherein one or more of the waxes have a melting point of at least about 40°C.

11. The razor cartridge of any preceding claim, wherein one or more of the waxes comprise a hydrocarbon-based wax, in particular a paraffin.

12. The razor cartridge of any preceding claim, wherein one or more of the waxes comprise an ester wax, in particular a carnauba wax.

13. The razor cartridge of any preceding claim, wherein the solid shaving aid composition comprises between about 1.0 wt.-% and about 5.0 wt.-%, and in particular between about 2.5 and about 4 wt.-%, of the one or more waxes, relative to the total weight of the solid shaving aid composition.

14. The razor cartridge of any one of claims 1 to 13, wherein the solid shaving composition is shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

15. A solid shaving aid composition having a composition as defined in any one of claims 1 to 14.
